# EUROPEAN PATENT APPLICATION

(11) **EP 1 142 602 A2**
(43) Date of publication of application: **10.10.2001**
(21) Application number: 01102586.3
(22) Date of filing: 06.02.2001
(51) Int. Cl.: A61M 16/00, A61M 16/01

(54) **Anaesthetic gas filter**

(30) Priority: 04.04.2000 SE 0001216
(71) Applicant: Siemens-Elema AB, 171 95 Solna (SE)
(72) Inventor: Pessala, Tom, 01100 Östersundom (FI)

(57) **Abstract**

An anaesthetic gas filter (2), devised for connection to an anaesthetic machine, for absorbing/adsorbing anaesthetic gas, is described. One problem with these filters is to determine when they need replacement without the leakage of any anaesthetic gas. This is solved with the anaesthetic gas filter according to the invention when an anaesthetic gas detector (22, 22B) is arranged in the filter. An early indication of the need to replace the filter is then provided.

## Description

The present invention relates to an anaesthetic gas filter according to the preamble to claim 1.

The description usually employs the term 'absorption of anaesthetic gas' and 'absorbent' for the material absorbing the anaesthetic gas. The corresponding effect and meaning can obviously be achieved with an adsorbent for adsorbing anaesthetic gas. So the concept 'absorption' in the description also includes 'adsorption' to prevent needless repetition.

US-3,867,936 describes an anaesthetic gas filter, intended for connection to the expiratory limb of an anaesthetic machine, for absorbing anaesthetic gas. The amount of anaesthetic gas absorbed can be estimated by weighing the filter. The filter is replaced with a new one when the weight increase reaches a given level.

This method has several disadvantages. A scale must be present if weighing is to take place during filter use. If weighing is only performed between usage occasions, the filter could become saturated during use, causing the leakage of anaesthetic gas.

EP-284 227 describes an apparatus and a process for recovering anaesthetic gas. An anaesthetic gas filter is placed on the outlet limb for expired gas in order to adsorb the anaesthetic gas. Here, the filter inlet is connected to an inlet line, and the filter outlet is connected to an outlet line. A shunt line runs parallel to the filter, and expired gas can pass either through the filter or through the shunt line depending on the setting of two valves. The filter is heated to prevent the condensation of water in the filter. A gas detector for anaesthetic gas is arranged downstream from the filter. The gas detector is connected to the anaesthetic machine. Detection of the presence of anaesthetic gas triggers an alarm on the anaesthetic machine to indicate that the filter is saturated and should be replaced. When a filter is replaced, the valves are reset to allow all gas to flow through the shunt line.

A major disadvantage of this design is that the alarm is not activated until anaesthetic gas has already begun leaking into atmosphere. In addition, two valves must first be reset to direct anaesthetic gas through the shunt line past the inlet and outlet lines each time the filter is replaced. The connections for the inlet line and outlet line must then be released. In the corresponding manner, the new filter must be connected to two lines, whereupon the valves must be reset to allow gas to pass the filter again. This means that filter replacement takes a relatively long time during which anaesthetic gas flows continuously into atmosphere.

One objective of the present invention is to achieve an anaesthetic gas filter which resolves the problems, in whole or part, associated with the known anaesthetic gas filters.

This objective is achieved according to the invention when the filter is devised as is evident from the characterising part of claim 1.

Advantageous embodiments and refinements are evident from the dependent claims of claim 1.

Arranging an anaesthetic gas detector in the filter provides the earliest possible indication that the filter is saturated and in need of replacement. Locating the detector in the filter also contributes to a simpler filter design employing only one connector to the anaesthetic machine (the other end of the filter can discharge residual gas straight into atmosphere.) This makes filter replacement much faster and radically reduces the amount of anaesthetic gas leaking into atmosphere each time a filter is replaced.

In one preferred embodiment, the filter according to the invention comprises a first absorption volume with a first absorbent and a second absorption volume with a second absorbent, separated by the anaesthetic gas detector. When the first absorbent is so saturated that leaking anaesthetic gas starts reaching the detector, a first indication is triggered showing that the filter's first absorbent is no longer absorbing/adsorbing anaesthetic gas. The second absorbent simultaneously begins absorbing the leaking anaesthetic gas. Thus, no anaesthetic gas escapes into atmosphere, even when the filter is not replaced immediately.

The absorbents can advantageously be separately replaceable, the second absorbent then being usable for a longer time since it does not need to adsorb gas for long periods.

In order to completely minimise the escape of anaesthetic gas, the filter can be equipped with flow channels that carry gas past the corresponding absorption volume when any absorbent is replaced. If, for example, the first absorbent is replaced, all gas is carried straight to the second absorbent which then absorbs all the anaesthetic gas.

The anaesthetic gas detector can consist of an indicator material that reacts with anaesthetic gas by changing colour or the like. The indicator material is placed in a visible position in the filter and provides a simple and distinct indication that the filter (or absorbent) is in need of replacement. This is particularly advantageous when anaesthetic machines are used at locations other than hospitals.

Alternately, the anaesthetic gas detector can consist of a sensor devised to identify anaesthetic gas. Sensors of this kind usually generate a signal when anaesthetic gas is detected. The signal can either be sent to an alarm unit in the anaesthetic machine or to a signal lamp or the equivalent on the filter.

Two embodiments of the anaesthetic gas machine will be described below in greater detail, referring to the figures.
FIG. 1 shows one embodiment of the anaesthetic gas filter according to the invention in longitudinal section and cross section.
FIG. 2 shows the anaesthetic filter according to FIG. 1 during the replacement of absorbent.
FIG. 3 shows a second embodiment of the anaesthetic gas filter according to the invention.

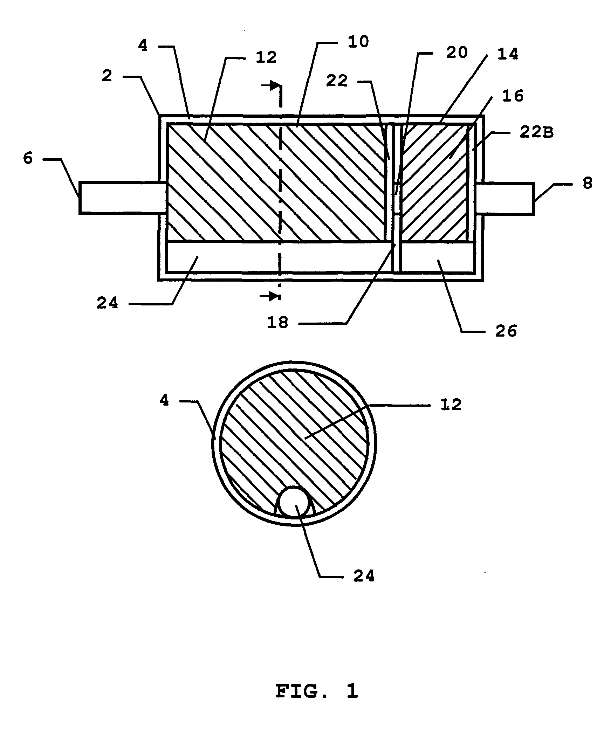

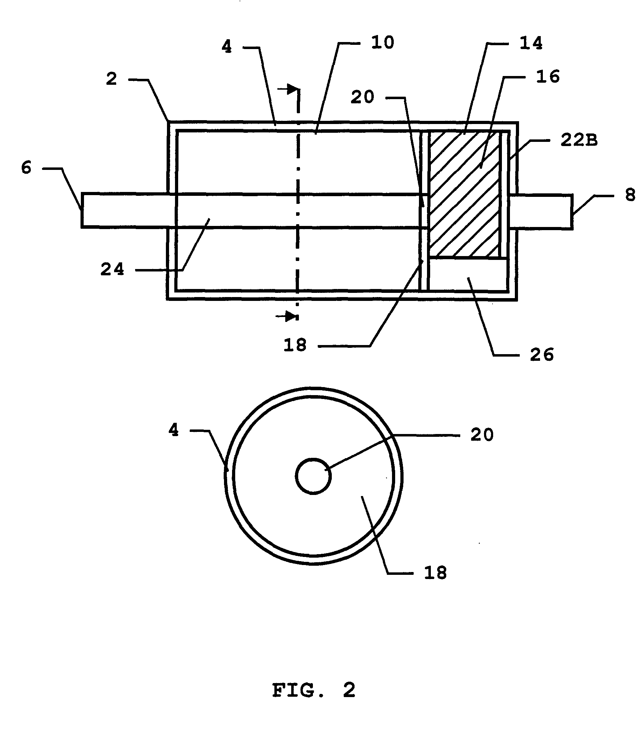

The following description generally applies to both FIG. 1 and FIG. 2. An anaesthetic gas filter 2 comprises an enclosure 4, an inlet 6 and an outlet 8. The inlet 6 is devised for connection to an anaesthetic machine in order to receive expired gas. The outlet 8 can be open to atmosphere or devised for connection to an evacuation line or the like.

Inside the enclosure 4, the filter 2 is divided into a first absorption volume 10, in which a first absorbent 12 is arranged, and a second absorption volume 14, in which a second absorbent 16 is arranged. A partition 18 separates the two absorption volumes 10, 14. A hole 20 in the partition 18 is arranged to allow the passage of gas.

An anaesthetic gas detector in the form of an indicator material 22 is arranged next to the first absorbent 12. The indicator material 22 is arranged so it is visible through the enclosure 4 and changes colour when it comes into contact with anaesthetic gas.

When the indicator material 22 changes colour, the first absorbent 12 is therefore deemed to be saturated and in need of replacement. The enclosure 4 is devised with hatches (not shown) providing access for replacement of absorbents 12, 16. The absorbents 12, 16 can advantageously have a gas-tight outer coating preventing gas from escaping through the hatches when the hatches are open.

When the first absorbent 12 is lifted out (as shown in FIG. 2), a first shunt line 24 automatically deploys and connects to the inlet 6 and the hole 20 in the partition 18. All gas then passes straight to the second absorption volume 14 and the second absorbent 16. Minimal leakage then occurs when the first absorbent 12 is replaced.

In the corresponding manner, a second shunt line 26 is arranged in the second absorption volume 14. When the second absorbent 16 is replaced, the second shunt line 26 automatically deploys to connect the hole 20 in the partition 18 to the outlet 8. The second shunt line 26 mainly has a function when the outlet 8 is connected to an evacuation line or the equivalent. Additional indicator material 22B can be arranged after the second absorbent 16 to show when the second absorbent 16 needs to be replaced.

The shunt lines 24, 26 can be spring-loaded or devised using some other known design in order to achieve automatic deployment. Alternately, valves can be arranged to automatically switch the gas through shunt lines when an absorbent is lifted out of the filter.

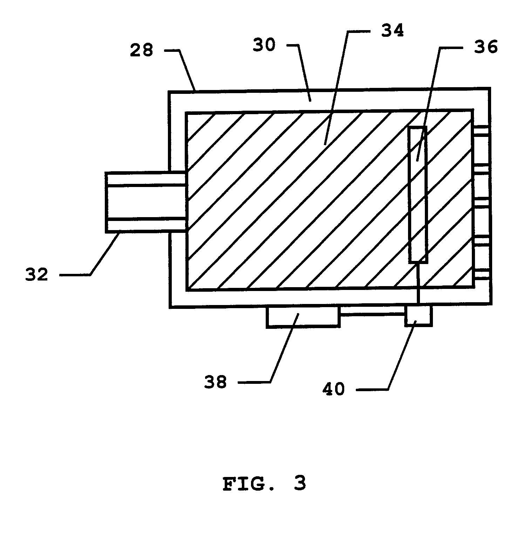

FIG. 3 shows a second embodiment of the anaesthetic gas filter according to the invention. The anaesthetic gas filter 28 consists of an enclosure 30 and an inlet 32 at one end for connection to an anaesthetic machine. The enclosure 30 is perforated at the other end to release unabsorbed gas into atmosphere.

An absorbent 34 is arranged in the enclosure to absorb anaesthetic gas. All known materials for absorbing anaesthetic gas can be used (individually or in various combinations). Activated charcoal and zeolites are just two examples of such materials.

An anaesthetic sensor 36 is arranged in the absorbent 34. The anaesthetic sensor 36 is advantageously placed close to the perforated part of the enclosure 30. When the anaesthetic gas sensor 36 senses the presence of anaesthetic, it generates a signal sent to an alarm indicator 38 on the enclosure 30. The alarm indicator 38 shows, with an optical signal, acoustic signal or both, that the filter needs replacement.

The anaesthetic sensor 36 and the alarm indicator 38 can advantageously consist of simpler components which can be discarded with the filter. They can also be transferred to the next filter or reused in other filters (the enclosure 30 can also be reused).

The signal from the anaesthetic sensor 36 can also be sent to the anaesthetic machine or to some other equipment (not shown) through a contact 40 for a signal line (not shown).

The illustrated embodiments can advantageously be combined in different ways, For example, the anaesthetic gas filter according to the first embodiment can be devised with an anaesthetic sensor instead of indicator material (and vice-versa for the anaesthetic gas filter according to the second embodiment). The anaesthetic gas filter according to the first embodiment can also be devised with a perforated enclosure instead of a connectable outlet (and vice-versa for the anaesthetic gas filter according to the second embodiment). Other combinations are obviously possible.

## Claims

1. An anaesthetic gas filter (2; 28), devised for connection to an anaesthetic machine, for absorption/adsorption of anaesthetic gas, **characterised in that** an anaesthetic gas detector (22, 22B; 36) is arranged in the filter.

2. The anaesthetic gas filter according to claim 1, **characterised in that** the filter comprises a first absorption volume (10) with a first absorbent (12) and a second absorption volume (14) with a second absorbent (16), the volumes are separated by the anaesthetic gas detector (22).

3. The anaesthetic gas filter according to claim 2, **characterised in that** each of the absorbents (12, 16) is arranged for individual replacement in the absorption volumes (10, 14).

4. The anaesthetic gas filter according to claim 3, **characterised in that** the filter is equipped with flow channels (24, 26) devised to carry gas past the corresponding absorption volume (10, 14) whenever any absorbent (12, 16) is replaced.

5. The anaesthetic gas filter according to any of claims 1-4, **characterised in that** the anaesthetic gas detector consists of an indicator material (22, 22B) which reacts with the anaesthetic gas and is visibly arranged in the filter.

6. The anaesthetic gas filter according to any of claims 1-4, **characterised in that** the anaesthetic gas detector consists of a sensor (36) devised to identify the anaesthetic gas.
